# EUROPÄISCHE PATENTSCHRIFT

(11) **EP 0 569 823 B1**
(45) Veröffentlichungstag und Bekanntmachung des Hinweises auf die Patenterteilung: **28.08.1996**
(21) Anmeldenummer: 93107215.1
(22) Anmeldetag: 04.05.1993
(51) Int. Cl.: C07C 229/62, C07C 227/16

(54) **Verfahren zur Herstellung von 2,5-Di-phenylamino-terephthalsäure und ihrer Dialkylester**
Process for the preparation of 2,5-di-phenylamino terephthalic acid and its dialkyl esters
Procédé de préparation de l'acide 2,5-di-phénylamino-térephtalique et ses esters dialcoyliques

(30) Priorität: 12.05.1992 DE 4215685
(43) Veröffentlichungstag der Anmeldung: 18.11.1993
(73) Patentinhaber: HOECHST AKTIENGESELLSCHAFT, 65926 Frankfurt am Main (DE)
(72) Erfinder: Arndt, Otto, Dr., W-6238 Hofheim/Ts. (DE)

(56) Entgegenhaltungen:
- EP-A- 0 057 873
- EP-A- 0 363 756
- EP-A- 0 373 959
- DE-A- 1 915 436
- US-A- 3 342 823
- CHEMICAL ABSTRACTS, Band 102, Seite 525, Zusammenfassung Nr. 5780p, Columbus, Ohio, US: M. P. DOYLE: "Rhodium (II)acetate catalyzed hydrocarbon oxidations by molecular oxygen"

## Beschreibung

Die vorliegende Erfindung betrifft ein in wirtschaftlicher Hinsicht und in Sachen Umweltschutz verbessertes Verfahren zur Herstellung von 2,5-Di-phenylamino-terephthalsäure und ihrer Dialkylester, insbesondere ihrer Methyl- und Ethylester.

Es ist bekannt, 2,5-Di-phenylamino-terephthalsäure und deren Dialkylester über ein mehrstufiges Verfahren herzustellen, indem man in einem ersten Schritt einen Bernsteinsäure-di-alkylester nach Art einer Dieckmann- bzw. doppelten Claisen-Kondensation zu dem entsprechenden 2,5-Dihydroxy-cyclohexadien-dicarbonsäure-(1,4)-dialkylester cyclisiert, diesen anschließend durch Kondensation mit einem primären Phenylamin (beispielsweise Anilin oder Toluidin) in Xylol oder Ethylbenzol oder deren Gemischen in Gegenwart einer aliphatischen Säure (beispielsweise Essigsäure) in den entsprechenden 2,5-Di-phenylamino-dihydro-(3,6)-terephthalsäure-dialkylester umzuwandeln, diesen durch Oxidation (Dehydrierung) in den korrespondierenden 2,5-Di-phenylamino-terephthalsäure-dialkylester überführt, diesen Ester anschließend mittels alkoholischer Natronlauge verseift und aus dem angefallenen Natriumsalz der 2,5-Di-phenylamino-terephthalsäure mittels einer Säure die freie 2,5-Di-phenylamino-terephthalsäure freisetzt.

Diese Art der Herstellung ist in der DE-OS-1 915 436 und DE-OS-3 834 747 (EP 0 363 756) beschrieben, wobei eine Reihe von Verfahrensparametern angegeben ist, wie Art des Lösungsmittels, Isolierung einzelner oder aller in der mehrstufigen Synthese anfallenden Produkte (hierzu zählen 2,5-Dihydroxy-cyclohexadien-dicarbonsäure-(1,4)-dialkylester, 2,5-Di-phenylamino-dihydro-(3,6)-terephthalsäure-di-alkylester, 2,5-Di-phenylamino-terephthalsäure-di-alkylester und 2,5-Di-phenylamino-terephthalsäure), ferner die Art der angewendeten Katalysatoren, gegebenenfalls mit Zusatzstoffen für die einzelnen Syntheseschritte, die zeitliche Reihenfolge von Oxidation und Verseifung (gleichzeitige Oxidation und Verseifung oder aufeinander folgend), Oxidationsmittel, wie Nitrobenzol und dessen Derivate, Chinone, Sauerstoff oder Jod, Aufarbeitung der verwendeten Hilfsstoffe, wie Lösungsmittel, Phenylamin (Folgeprodukt von Nitrobenzol), Katalysatoren und sonstige Zusatzstoffe.

Aus Gründen des Umweltschutzes ist es wenig sinnvoll, die als Oxidationsmittel für die Dehydrierungsstufe statt Luft bzw. Sauerstoff empfohlenen Nitroaromaten (Nitrobenzol oder Derivate wie Nitrobenzol-m-sulfonsäure) zu verwenden. Ein Stoff wie Nitrobenzol sollte allenfalls als Baustein in Synthesen, nicht jedoch als Hilfsstoff verwendet werden, da seine Folgeprodukte, wie Anilin, Azo- oder Azoxybenzol, unter hohem Aufwand entfernt werden müssen und sich wegen der dabei unvermeidbaren starken Verunreinigungen nicht wieder verwerten lassen. Sie fallen also als die Umwelt belastende Abfallstoffe, die entweder das Abwasser belasten oder deponiert werden müssen, an.

Verfahren, die in der Stufe der Oxidation Luft in Verbindung mit Zusatzstoffen (Chinone und deren Derivate, wie Sulfonsäuren und Chloranil, oder quarternäre Ammoniumsalze) (EP-57 873 und EP-363 576), als Oxidationsmittel verwenden, sind ebenfalls aus Gründen des Umweltschutzes als nachteilig zu bewerten, da diese Zusatzstoffe im allgemeinen in das Abwasser gelangen und in einigen Fällen stark wassergefährdend wirken. Eine Rückgewinnung der Zusatzstoffe ist in keinem der vorstehend genannten Verfahren vorgesehen.

Das Verfahren gemäß DE-OS-1 915 436 weist eine Reihe von Nachteilen auf. Zum einen fällt das in der Oxidationsstufe gebildete 2,5-Di-phenylamino-terephthalsäure-dialkylester-Rohprodukt nach Filtration als ein Xylol enthaltendes Gemisch an, aus dem das Xylol durch Wäsche, z. B. mit Aceton, entfernt werden muß. Dieses Gemisch aus Aceton und Xylol muß gesondert aufgearbeitet werden. Zum anderen ist offensichtlich noch eine zusätzliche Umkristallisation des Rohproduktes erforderlich, um Zu einem ausreichend sauberen Produkt zu gelangen. Wegen der Löslichkeit des Esters in Xylol muß vor der Filtration auf eine Temperatur < 15 °C abgekühlt werden, was einen nicht unerheblichen technischen Aufwand erfordert. Darüber hinaus befindet sich der größte Teil der Nebenprodukte im abgefilterten Xylol. Dies führt dazu, daß bei der destillativen Rückgewinnung des Xylols ein teerartiger Sumpf anfällt, der nur durch Verbrennung entsorgt werden kann. Im Verlauf der Oxidation bildet sich schließlich eine Verunreinigung, die sich als ein dünner, dunkelbrauner Belag eines Feststoffes an der Wand des Reaktionsgefäßes und auf dem Rührer abscheidet.

Zusammenfassend ist festzustellen, daß sowohl die Isolierung des 2,5-Di-phenylarnino-terephthalsäure-dialkylesters mit einem erheblichen technischen Aufwand verbunden ist, als auch bezüglich der Entsorgung der Abfallprodukte Nachteile in Sachen Ökologie in Kauf genommen werden müssen.

Aufgrund der vorangegangenen Erläuterungen besteht ein Bedarf nach einem Verfahren, das die vorstehend geschilderten Nachteile vermeidet und sich zudem einfach und unter Zuhilfenahme leicht zugänglicher Hilfsstoffe durchführen laßt.

Diese Aufgabe wird gelöst durch ein Verfahren zur Herstellung von 2,5-Di-phenylamino-terephthalsäure und ihrer Dialkylester der allgemeinen Formel 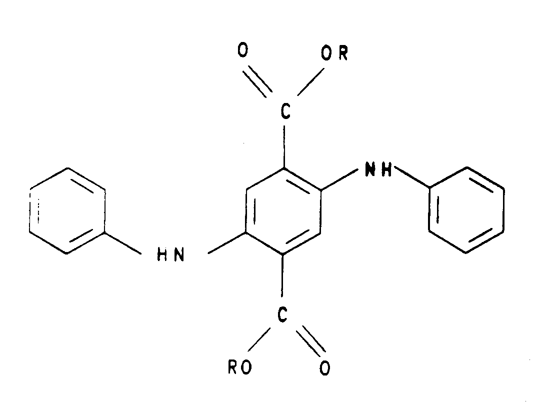
worin R für ein Wasserstoffatom oder eine Methyl- oder eine Ethylgruppe steht, durch Umsetzung eines Bernsteinsäuredialkylesters mit Natriumalkoholat in Xylol, Behandlung des gebildeten 2,5-Dihydroxy-cyclohexadien-di-carbonsäure-(1,4)-dialkylesters mit Säure und Anilin, Dehydrierung des gebildeten 2,5-Di-phenylamino-dihydro-(3,6)-terephthalsäuredialkylesters mittels Sauerstoff, gegebenenfalls Verseifung des gebildeten 2,5-Di-phenylamino-terephthalsäuredialkylesters mit methanolischer Natronlauge und Freisetzung der 2,5-Di-phenylamino-terephthalsäure aus dem gebildeten Di-Natriumsalz der 2,5-Di-phenylamino-terephthalsäure, dadurch gekennzeichnet, daß man den 2,5-Di-phenylamino-dihydro-(3,6)-terephthalsäuredialkylester in Gegenwart eines Alkalimetallions und/oder Erdalkalimetallions mit reinem Sauerstoff umsetzt.

Ein Vorteil des Verfahrens besteht darin, daß die Verwendung von Sauerstoff lediglich zu einem Reaktionsprodukt, nämlich Wasser, führt. Ferner ist es möglich, in allen Einzelschritten der mehrstufigen Synthese ein einziges Lösungsmittel zu verwenden. Als geeignet hat sich beispielsweise Xylol erwiesen.

Ein weiterer, wichtiger Vorteil des Verfahrens besteht darin, daß auch der im Reaktionsgefäß an der Wandung und auf dem Rührer abgeschiedene dunkelbraune Belag nicht mehr auftritt.

Das erfindungsgemäße Verfahren wirkt sich auch günstig auf die Qualität der freien 2,5-Di-phenylamino-terephthalsäure aus. Üblicherweise enthält die nach dem Verfahren des Standes der Technik hergestellte freie 2,5-Di-phenylamino-terephthalsäure, wie HPLC (high performance liquid chromatography) und HPTLC (high performance thinlayer chromatography) Untersuchungen belegen, in einem bestimmten Bereich noch Verunreinigungen, die etwa zwischen 0,5 bis 1,0 %, bezogen auf Gesamtprodukt, betragen. Überraschenderweise wird diese Verunreinigung, insbesondere eine ihrer Komponenten, durch das erfindungsgemäße Verfahren erheblich verringert.

Der zur erfolgreichen Durchführung des erfindungsgemäßen Verfahrens benötigte Bedarf an Alkalimetallionen oder Eralkalimetallionen ist vergleichweise gering und entspricht katalytischen Mengen. Im allgemeinen genügt es, 0,2 bis 12,0, insbesondere 0,8 bis 6,0 Mol % Alkalimetallionen oder Eralkalimetallionen, bezogen auf 2,5-Di-phenylamino-dihydro-(3,6)-terephthalsäuredialkylester einzusetzen.

Bedingt durch die geringe Menge und durch die Art der verwendeten Zusatzstoffe (Alkalimetallionen oder Erdalkalimetallionen) läßt sich die Belastung des Abwassers niedrig halten und das Verfahren umweltfreundlich gestalten.

Die Nebenprodukte verteilen sich auf die wäßrige Mutterlauge und auf das methanolische Waschfiltrat vorteilhafterweise derart, daß der Anteil im Abwasser biologisch zu über 98 % abbaubar ist (gemessen als Eliminierbarkeit nach DIN 38412-L25), der Anteil im Methanol nur noch ca. 33 bis ca. 50 % der nach dem Verfahren der DOS 195436 anfallenden Menge beträgt, somit deutlich weniger organisches Produkt durch Verbrennung entsorgt werden muß.

Es hat sich als vorteilhaft herausgestellt, als Alkalimetallion Na⁺ oder K⁺ und als Erdalkalimetallion Mg²⁺, Ca²⁺ oder Sr²⁺ zu verwenden.

Besonders einfach gestaltet sich das Verfahren, indem man die Alkalimetallionen oder Erdalkalimetallionen in Form von Alkalimetalloxiden oder Erdalkalimetalloxiden, Alkalimetallsalzen oder Erdalkalimetallsalzen, insbesondere von Carbonaten oder Carbonsäuresalzen einsetzt. Besonders bewährt hat sich die Verwendung von Alkalimetallsalzen oder Erdalkalimetallsalzen aliphatischer Carbonsäuren, insbesondere aliphatischer Monocarbonsäuren mit 2 bis 8 Kohlenstoffatomen. Besonders einfach ist der Gebrauch von Alkalimetallacetaten oder Erdalkalimetallacetaten.

Es hat sich besonders bewährt, die Oxidation in einem geschlossenen System, d. h. unter dem sich jeweils einstellenden Eigendruck, durchzuführen. Es ist allerdings auch möglich, die Oxidation in einem offenen System ablaufen zu lassen.

Üblicherweise führt man die Oxidation bei 95 bis 110, insbesondere 98 bis 103°C durch. Die Oxidation läßt sich sowohl unter Atmosphärendruck als auch unter erhöhtem Druck ausführen. Geeignete Drücke sind 0,1 bis 1,0, insbesondere 0,1 bis 0,5 MPa. Die Oxidation läßt sich jedoch auch bei Drücken oberhalb 1,0 MPa ausführen, wobei ein entsprechend höherer technischer Aufwand erforderlich ist. Als Lösungsmittel haben sich o-Xylol, m-Xylol oder Ethylbenzol, insbesondere technisches Xylol bewährt. Technisches Xylol ist das im Handel befindliche Gemisch isomerer Xylole, das zusätzlich Ethylbenzol enthält.

Nach einer bevorzugten Verfahrensvariante führt man zunächst die Oxidation unter Verwendung von Xylol als Lösungsmittel durch, entfernt anschließend nach Abschluß der Oxidation das Xylol durch Wasserdampfdestillation, filtriert den gebildeten 2,5-Di-phenylamino-terephthalsäure-dialkylester ab, wäscht den abfiltrierten Ester mit einem aliphatischen Alkohol, insbesondere Methanol, Ethanol oder Propanol, bevorzugt Methanol, und trocknet anschließend den durch den Waschvorgang gereinigten 2,5-Di-phenylamino-terephthalsäure-dialkylester.

2,5-Di-phenylamino-terephthalsäure und ihre Ester sind, wie aus der DE-OS-1 915 436 hervorgeht, wichtige Ausgangsstoffe für die Herstellung von lumineszierenden Massen.

Die erfindungsgemäß hergestellte 2,5-Di-phenylamino-terephthalsäure und ihre Ester eignen sich besonders zur Herstellung von Chinacridonen.

Die nachfolgenden Beispiele belegen die Erfindung, ohne sie zu beschränken.

### Experimenteller Teil

### Beispiel 1

### Herstellung von 2,5-Di-phenylamino-cyclohexadien-dicarbonsäure-(1,4)-dimethylester

In einer Glasapparatur, die mit einem Rührer, einem Rückflußkühler, einem Wasserabscheider zum Auskreisen von Wasser und einem Anschluß zum Einleiten von Stickstoff bestückt ist, werden 500 Teile Xylol technischer Qualität (handelsübliches Isomerengemisch, das zusätzlich Ethylbenzol enthält) und 85 Teile Propionsäure (etwa 99 %ig) vorgelegt. Unter Stickstoffschutz werden 114 Teile 2,5-Di-hydroxy-cyclohexadien-dicarbonsäure-(1,4)-dimethylester (etwa 97 bis 98 %ig) eingetragen. Anschließend gibt man bei ca. 75 - 90°C, weiterhin unter Stickstoff, 112 Teile Anilin unter Rühren hinzu. Man erhitzt das Gemisch der Einsatzstoffe von 90 auf etwa 98 bis 100 °C in etwa 3 Stunden unter Durchleiten von Stickstoff und läßt zur Vervollständigung der Reaktion (quantitative Abscheidung der theoretischen Menge Reaktionswasser) noch 45 Minuten unter weiterem Durchleiten von Stickstoff nachreagieren.

### Herstellung von 2,5-Di-phenylamino-terephthalsäure-dimethylester

Man setzt dem 2,5-Di-phenylamino-cyclohexadien-dicarbonsäure-(1,4)-dimethylester enthaltenden Gemisch 1,3 Teile Magnesiumacetat-Tetrahydrat zu. Anstelle von Stickstoff leitet man nunmehr einen Strom reinen Sauerstoff (3 Liter/Stunde) über ein zylindrisch erweitertes Zuführungsrohr, das zur besseren Verteilung des Sauerstoffstroms mit einem Netz aus Edelstahl verbunden ist, ein. Man erhitzt auf eine Temperatur von etwa 98 bis 100 °C. Durch die nun einsetzende Oxidation (Dehydrierung) wird der Cyclohexadien-Ring zu einem Benzol-Ring aufoxidiert. Hierbei entsteht erneut Reaktionswasser, das durch einen über das Einsatzgemisch geleiteten Stickstoffstrom (12 Liter/Stunde) mitgenommen und anschließend nach Kondensation im Rückflußkühler über den Wasserabscheider aus dem Reaktionssystem entfernt wird. Die Oxidation ist, erkennbar an der Beendigung der Wasserabspaltung, nach etwa 2,5 Stunden beendet. Eine zusätzliche Kontrolle der Umsetzung erfolgt durch Flüssigkeitschromatographie (HPLC). Man erhält den 2,5-Di-phenylamino-terephthalsäure-dimethylester in Form einer dunkelgefärbten Lösung.

Anschließend entfernt man durch Einleiten von Wasserdampf (Wasserdampfdestillation) das als Lösungsmittel eingesetzte Xylol, wobei der 2,5-Di-phenylamino-terephthalsäure-dimethylester als Feststoff ausfällt. Man filtriert mit Hilfe einer Nutsche bei 80 °C, wäscht mit siedend heißem Wasser und bläst etwa 15 Minuten lang Wasserdampf durch das auf der Nutsche befindliche Dimethylester-Rohprodukt. Anschließend wäscht man dieses Produkt mit etwa 500 - 700 Teilen Methanol bei 50 °C und erhält nach Trocknen im Trockenofen 173,1 Teile 2,5-Di-phenylamino-terephthalsäure-dimethylester (entsprechend einer Ausbeute von 92,0 %, bezogen auf eingesetzten 2,5-Di-hydroxy-cyclohexadien-dicarbonsäure-(1,4)-dimethylester) mit einem Schmelzpunkt von etwa 160 - 163 °C.

Man arbeitet das bei der Wäsche verwendete Methanol destillativ auf und erhält einen teerartigen Rückstand mit 14,1 Teilen Feststoff, der durch Verbrennen entsorgt wird.

Das bei der Wasserdampfdestillation von Xylol und bei der Filtration des rohen Dimethylesters anfallende gesamte Abwasser hat einen Gehalt von ca. 50 Teilen organischen Kohlenstoff und weist eine CSB-Eliminierung von über 98 % auf.

### Herstellung von 2,5-Di-phenylamino-terephthalsäure

Man setzt den 2,5-Di-phenylamino-terephthalsäure-dimethylester unmittelbar nach Wäsche mit Methanol, unter Verzicht auf eine Trocknung, direkt ein und verseift ihn mit einem Gemisch aus Methanol, Wasser und Natronlauge in einem Rührautoklav bei 106 °C innerhalb von 3 Stunden zu dem entsprechenden Di-natriumsalz. Die alkalische Lösung des Reaktionsgemisches wird unter Stickstoffschutz filtriert und anschließend destillativ von Methanol befreit. Aus der nahezu methanolfreien Lösung wird die 2,5-Di-phenylamino-terephthalsäure mit konzentrierter Salzsäure gefällt.

Man erhält 154,7 Teile 2,5-Di-phenylamino-terephthalsäure (entsprechend einer Ausbeute von 88,8 %, bezogen auf 2,5-Di-hydroxy-cyclohexadien-dicarbonsäure-(1,4)-dimethylester) mit einem Schmelzpunkt von 319 °C (Zersetzung).

Das Chromatogramm der Reverse-Phase-HPLC zeigt im unterhalb der 2,5-Di-phenylamino-terephthalsäure liegenden Retentionsbereiches einen Peak einer Komponente mit einer Retentionszeit von 5,0 Minuten entsprechend einer Intensität von 0,196 Flächenprozent.

### Beispiel 2

Man arbeitet wie in Beispiel 1 angegeben, setzt jedoch statt Magnesiumacetat-Tetrahydrat 0,337 Teile Calciumacetat (94 %ig) zu.
Man erhält 172,4 Teile 2,5-Di-phenylamino-terephthalsäure-dimethylester (entsprechend einer Ausbeute von 91,6 %, bezogen auf eingesetzten 2,5-Di-hydroxy-cyclohexadien-dicarbonsäure-(1,4)-dimethylester) mit einem Schmelzpunkt von etwa 159 bis 162 °C.

Nach destillativer Aufarbeitung des bei der Wäsche des Dimethylester anfallenden Methanol fällt ein teerartiger Rückstand mit 15,9 Teilen Feststoff an.

Nach Verseifen und Ansäuern erhält man 153,0 Teile 2,5-Di-phenylamino-terephthalsäure (entsprechend einer Ausbeute von 87,9 %, bezogen auf 2,5-Di-hydroxy-cyclohexadien-dicarbonsäure-(1,4)-dimethylester) mit einem Schmelzpunkt von 318°C (Zersetzung).

Das Chromatogramm der Reverse-Phase-HPLC zeigt im unterhalb der 2,5-Di-phenylamino-terephthalsäure gelegenen Retentionsbereich den Peak der Komponente entsprechend einer Intensität von 0,135 Flächenprozent.

### Beispiel 3

Man arbeitet wie in Beispiel 1 angegeben, setzt jedoch statt Magnesiumacetat-Tetrahydrat 0,595 Teile Kaliumacetat (99 %ig) zu. Man erhält 173,1 Teile 2,5-Di-phenylamino-terephthalsäure-dimethylester (entsprechend einer Ausbeute von 92,0 %, bezogen auf eingesetzten 2,5-Di-hydroxy-cyclohexadien-dicarbonsäure-(1,4)-dimethylester) mit einem Schmelzpunkt von etwa 158 bis 162 °C.

Nach destillativer Aufarbeitung des bei der Wäsche des Dimethylester anfallenden Methanols fällt ein teerartiger Rückstand mit etwa 16,5 Teilen Feststoff an.

Nach Verseifen und Ansäuern erhält man 156,6 Teile 2,5-Di-phenylamino-terephthalsäure (entsprechend einer Ausbeute von 89,9 %, bezogen auf 2,5-Di-hydroxy-cylcohexadien-dicarbonsäure-(1,4)-dimethylester) mit einem Schmelzpunkt von 316°C (Zersetzung).

Das Chromatogramm der Reverse-Phase-HPLC zeigt im unterhalb der 2,5-Di-phenylamino-terephthalsäure liegenden Retentionsbereich den Peak der Komponente mit der Retentionszeit von 5,0 Minuten entsprechend einer Intensität von 0,176 Flächenprozent.

### Beispiel 4

Man arbeitet wie in Beispiel 1 angegeben, setzt jedoch statt Magnesiumacetat 5,0 Teile Natriumacetat (99 %ig) (= 12 Mol %) zu. Man erhält 173,2 Teile 2,5-Di-phenylamino-terephthalsäure-dimethylester (entsprechend einer Ausbeute von 90,2 % bezogen auf eingesetzten 2,5-Di-hydroxy-cyclohexadien-dicarbonsäure-(1,4)-dimethylester) mit einem Schmelzpunkt von etwa 158 - 162 °C.

Nach destillativer Aufarbeitung des bei der Wäsche des Dimethylesters anfallenden Methanols fällt ein teerartiger Rückstand mit 16,8 Teilen Feststoff an.

Nach Verseifen und Ansäuern erhält man 156,0 Teile 2,5-Di-phenylamino-terephthalsäure (entsprechend einer Ausbeute von 89,6 % bezogen auf 2,5-Di-hydroxy-cyclohexadien-dicarbonsäure-(1,4)-dimethylester) mit einem Schmelzpunkt von 318 °C (Zersetzung).

Das Chromatogramm der Reverse-Phase-HPLC zeigt im unterhalb der 2,5-Di-phenylamino-terephthalsäure gelegenen Retentionsbereich den Peak der Komponente entsprechend einer Intensität von 0,122 Flächenprozent.

### Vergleichsbeispiel 1

Man arbeitet, wie in Beispiel 1 angegeben, jedoch ohne Zusatz von Magnesiumacetat-Tetrahydrat. Man erhält 176,0 Teile 2,5-Di-phenylamino-terephthalsäure-dimethylester (entsprechend einer Ausbeute von 93,5 %, bezogen auf eingesetzten 2,5-Di-hydroxy-cyclohexadien-dicarbonsäure-(1,4)-dimethylester) mit einem Schmelzpunkt von etwa 159 bis 161 °C. In der Stufe der Oxidation bildet sich an der Wand des Glasapparates und am Rührer ein dünner, dunkelbrauner Belag eines Feststoffes.

Nach destillativer Aufarbeitung des bei der Wäsche des rohen Dimethylesters anfallenden Methanols fällt ein teerartiger Rückstand mit 11,8 Teilen Feststoff an.

Nach Verseifen und Ansäuern erhält man 158,0 Teile 2,5-Di-phenylamino-terephthalsäure (entsprechend einer Ausbeute von 90,7 %, bezogen auf 2,5-Di-hydroxy-cyclohexadien-dicarbonsäure-(1,4)-dimethylester) mit einem Schmelzpunkt von etwa 316°C (Zersetzung).

Das Chromatogramm der Reverse-Phase-HPLC zeigt den Peak der Komponente mit der Retentionszeit von 5 Minuten entsprechend einer Intensität von 0,527 Flächenprozent.

## Patentansprüche

1. Verfahren zur Herstellung von 2,5-Di-phenylamino-terephthalsäure und ihrer Dialkylester der allgemeinen Formel 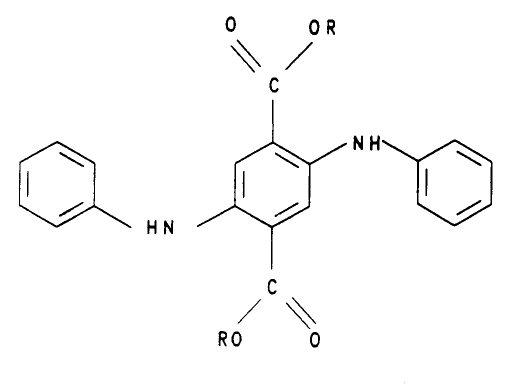 worin R für ein Wasserstoffatom oder eine Methyl- oder eine Ethylgruppe steht, durch Umsetzung eines Bernsteinsäuredialkylesters mit Natriumalkoholat in Xylol, Behandlung des gebildeten 2,5-Dihydroxy-cyclohexadien-di-carbonsäure-(1,4)-dialkylesters mit Säure und Anilin, Dehydrierung des gebildeten 2,5-Di-phenylamino-dihydro-(3,6)-terephthalsäuredialkylesters mittels Sauerstoff, gegebenenfalls Verseifung des gebildeten 2,5-Di-phenylamino-terephthalsäuredialkylesters mit methanolischer Natronlauge und Freisetzung der 2,5-Di-phenylamino-terephthalsäure aus dem gebildeten Di-Natriumsalz der 2,5-Di-phenylamino-terephthalsäure, dadurch gekennzeichnet, daß man den 2,5-Di-phenylamino-dihydro-(3,6)-terephthalsäuredialkylester in Gegenwart eines Alkalimetallions und/oder Erdalkalimetallions mit reinem Sauerstoff umsetzt.

2. Verfahren nach Anspruch 1, dadurch gekennzeichnet, daß man 0,2 bis 12,0, insbesondere 0,8 bis 6,0 Mol % Alkalimetallionen oder Erdalkalimetallionen, bezogen auf 2,5-Di-phenylamino-dihydro-(3,6)-terephthalsäuredialkylester, einsetzt.

3. Verfahren nach Anspruch 1 oder 2, dadurch gekennzeichnet, daß man als Alkalimetallion Na⁺ oder K⁺ und als Erdalkalimetallion Mg²⁺, Ca²⁺ oder Sr²⁺ einsetzt.

4. Verfahren nach einem oder mehreren der Ansprüche 1 bis 3, dadurch gekennzeichnet, daß man Alkalimetalloxide oder Erdalkalimetalloxide, Alkalimetallsalze oder Erdalkalimetallsalze, insbesondere deren Carbonate oder Carbonsäuresalze, einsetzt.

5. Verfahren nach einem oder mehreren der Ansprüche 1 bis 4, dadurch gekennzeichnet, daß man Alkalimetallsalze oder Erdalkalimetallsalze von aliphatischen Carbonsäuren, insbesondere aliphatischen Monocarbonsäuren mit 2 bis 8 Kohlenstoffatomen, einsetzt.

6. Verfahren nach einem oder mehreren der Ansprüche 1 bis 5, dadurch gekennzeichnet, daß man Alkalimetallacetate oder Erdalkalimetallacetate einsetzt.

7. Verfahren nach einem oder mehreren der Ansprüche 1 bis 6, dadurch gekennzeichnet, daß man die Oxidation in einem geschlossenen System durchführt.

8. Verfahren nach einem oder mehreren der Ansprüche 1 bis 7, dadurch gekennzeichnet, daß man die Oxidation bei 95 bis 110, insbesondere 98 bis 103°C durchführt.

9. Verfahren nach einem oder mehreren der Ansprüche 1 bis 8, dadurch gekennzeichnet, daß man die Oxidation unter einem Druck von 0,1 bis 1,0, insbesondere 0,1 bis 0,5 MPa durchführt.

10. Verfahren nach einem oder mehreren der Ansprüche 1 bis 9, dadurch gekennzeichnet, daß man Xylol als Lösungsmittel verwendet.

11. Verfahren nach einem oder mehreren der Ansprüche 1 bis 10, dadurch gekennzeichnet, daß man das Lösungsmittel, insbesondere Xylol, nach Abschluß der Oxidation durch Wasserdampfdestillation entfernt, den anfallenden 2,5-Di-phenylamino-terephthalsäuredialkylester abfiltriert, mit einem aliphatischen Alkohol, insbesondere Methanol, Ethanol, Propanol, bevorzugt Methanol, wäscht und trocknet.

## Claims

1. A process for the preparation of 2,5-di-phenylamino-terephthalic acid or one of its dialkyl esters of the formula 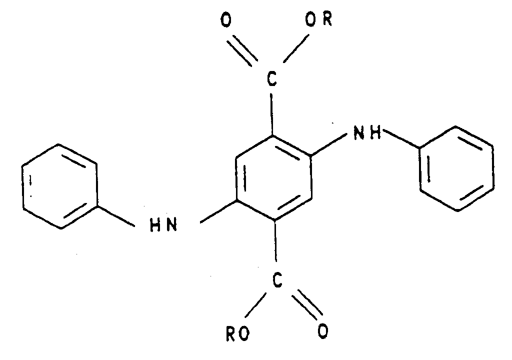 in which R is a hydrogen atom or a methyl or an ethyl group by reaction of a succinic acid dialkyl ester with a sodium alcoholate in xylene, treatment of the resulting 2,5-dihydroxy-cyclohexadiene-1,4-di-carboxylic acid dialkyl ester with acid and aniline, dehydrogenation of the resulting 2,5-di-phenylamino-dihydro-(3,6)-terephthalic acid dialkyl ester by means of oxygen, hydrolysis if desired of the resulting 2,5-di-phenylamino-terephthalic acid dialkyl ester with methanolic sodium hydroxide solution and liberation of 2,5-di-phenylamino-terephthalic acid from the 2,5-di-phenylamino-terephthalic acid di-sodium salt formed, which comprises reacting the 2,5-di-phenylamino-dihydro-(3,6)-terephthalic acid dialkyl ester with pure oxygen in the presence of an alkali metal ion and/or alkaline earth metal ion.

2. The process as claimed in claim 1, wherein 0.2 to 12.0, in particular 0.8 to 6.0 mol% of alkali metal ions or alkaline earth metal ions, based on 2,5-di-phenylamino-dihydro-(3,6)-terephthalic acid dialkyl ester, is employed.

3. The process as claimed in claim 1 or 2, wherein Na⁺ or K⁺ is employed as the alkali metal ion and Mg²⁺, Ca²⁺ or Sr²⁺ is employed as the alkaline earth metal ion.

4. The process as claimed in one or more of claims 1 to 3, wherein an alkali metal oxide or alkaline earth metal oxide, alkali metal salt or alkaline earth metal salt, in particular a carbonate or carboxylic acid salt thereof, is employed.

5. The process as claimed in one or more of claims 1 to 4, wherein an alkali metal salt or alkaline earth metal salt of an aliphatic carboxylic acid, in particular of an aliphatic monocarboxylic acid having 2 to 8 carbon atoms, is employed.

6. The process as claimed in one or more of claims 1 to 5, wherein an alkali metal acetate or alkaline earth metal acetate is employed.

7. The process as claimed in one or more of claims 1 to 6, wherein the oxidation is carried out in a closed system.

8. The process as claimed in one or more of claims 1 to 7, wherein the oxidation is carried out at 95 to 110, in particular 98 to 103°C.

9. The process as claimed in one or more of claims 1 to 8, wherein the oxidation is carried out under a pressure of 0.1 to 1.0, in particular 0.1 to 0.5 MPa.

10. The process as claimed in one or more of claims 1 to 9, wherein xylene is used as solvent.

11. The process as claimed in one or more of claims 1 to 10, wherein the solvent, in particular xylene, is removed by steam distillation after the oxidation has been concluded, and the 2,5-di-phenylamino-terephthalic acid dialkyl ester obtained is filtered off, washed with an aliphatic alcohol, in particular methanol, ethanol or propanol, preferably methanol, and dried.

## Revendications

1. Procédé pour la préparation d'acide 2,5-di-phénylamino-téréphtalique et de ses esters dialkyliques de formule générale 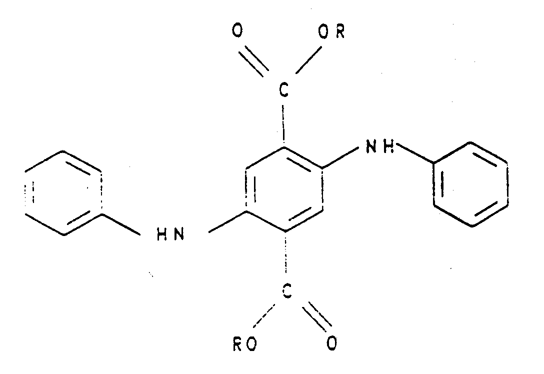 dans laquelle R représente un atome d'hydrogène ou un groupe méthyle ou un groupe éthyle, par réaction d'un ester dialkylique d'acide succinique avec un alcoolate de sodium dans du xylène, le traitement du 2,5-dihydroxy-cyclohexadiéne-dicarboxylate de (1,4)-dialkyle, formé par un acide et l'aniline, la déshydrogénation du 2,5-di-phénylamino-dihydro-(3,6)-téréphtalate de dialkyle formé par l'oxygène, éventuellement la saponification du 2,5-di-phényl-amino-téréphtalate de dialkyle par une lessive de soude méthanolée et la libération de l'acide 2,5-di-phénylamino-téréphtalique à partir du sel disodique de l'acide 2,5-di-phénylamino-téréphtalique formé, caractérisé en ce qu'on fait réagir le 2,5-di-phénylamino-dihydro-(3,6)-téréphtalate de dialkyle, en présence d'un ion de métal alcalin et/ou de métal alcalino-terreux, avec l'oxygène pur.

2. Procédé selon la revendication 1 caractérisé en ce qu'on utilise de 0,2 à 12,0, plus particulièrement de 0,8 à 6,0% en mole d'ions de métaux alcalins ou de métaux alcalino-terreux par rapport au 2,5-di-phénylamino-dihydro-(3,6)-téréphtalate de dialkyle.

3. Procédé selon la revendication 1 ou 2, caractérisé en ce qu'on utilise comme ion de métal alcalin, Na⁺ ou K⁺, et comme ion de métal alcalino-terreux Mg²⁺, Ca²⁺ ou Sr²⁺.

4. Procédé selon une ou plusieurs des revendications 1 à 3, caractérisé en ce qu'on utilise des oxydes de métaux alcalins ou des oxydes de métaux alcalino-terreux, des sels de métaux alcalins ou des sels de métaux alcalino-terreux, plus particulièrement leurs carbonates ou leurs sels d'acides carboxyliques.

5. Procédé selon une ou plusieurs des revendications 1 à 4, caractérisé en ce qu'on utilise les sels de métaux alcalins ou les sels de métaux alcalino-terreux d'acides carboxyliques aliphatiques plus particulièrement d'acides monocarboxyliquse aliphatiques avec 2 à 8 atomes de carbone.

6. Procédé selon une ou plusieurs des revendications 1 à 5, caractérisé en ce qu'on utilise des acétates de métaux alcalins ou les acétates de métaux alcalino-terreux.

7. Procédé selon une ou plusieurs des revendications 1 à 6, caractérisé en ce qu'on met en oeuvre l'oxydation dans un système fermé.

8. Procédé selon une ou plusieurs des revendications 1 à 7, caractérisé en ce qu'on met en oeuvre l'oxydation à une température de 95 à 110°C, plus particulièrement de 98 à 103°C.

9. Procédé selon une ou plusieurs des revendications 1 à 8, caractérisé en ce qu'on met en oeuvre l'oxydation à une pression de 0,1 à 1,0, plus particulièrement de 0,1 à 0,5 MPa.

10. Procédé selon une ou plusieurs des revendications 1 à 9, caractérisé en ce qu'on utilise le xylène comme solvant.

11. Procédé selon une ou plusieurs des revendications 1 à 10, caractérisé en ce qu'on élimine le solvant, plus particulièrement le xylène, après avoir terminé l'oxydation, par l'entraînement à la vapeur, on sépare par filtration le 2,5-di-phényl-amino-téréphtalate de dialkyle formé, on lave avec un alcool aliphatique, plus particulièrement le méthanol, l'éthanol, le propanol, notamment le méthanol, et on sèche.
